# EUROPÄISCHE PATENTSCHRIFT

(11) **EP 2 637 736 B1**
(45) Veröffentlichungstag und Bekanntmachung des Hinweises auf die Patenterteilung: **22.04.2015**
(21) Anmeldenummer: 11813383.4
(22) Anmeldetag: 07.11.2011
(51) Int. Cl.: A61N 1/05, B29D 30/10, B29D 30/16

(54) **ELEKTRODE ZUR INTRAOPERATIVEN NERVENSTIMULATION**
ELECTRODE FOR INTRAOPERATIVE NERVE STIMULATION
ÉLECTRODE POUR STIMULATION NERVEUSE PEROPÉRATOIRE

(30) Priorität: 11.11.2010 DE 102010060497
(43) Veröffentlichungstag der Anmeldung: 18.09.2013
(73) Patentinhaber: Dr. Langer Medical GmbH, 79183 Waldkirch (DE)
(72) Erfinder: LANGER, Andreas, 79183 Waldkirch (DE)
(74) Vertreter: Hennicke, Ernst Rüdiger
(86) Internationale Anmeldenummer: PCT/IB2011/054960
(87) Internationale Veröffentlichungsnummer: WO 2012/063189

(56) Entgegenhaltungen:
- WO-A1-2007/021535
- US-A1- 2007 129 780

## Beschreibung

Die Erfindung betrifft eine Elektrode zur intraoperativen Stimulation des Nervus vagus mit einem streifenförmig ausgebildeten Elektrodenträger aus elastischem Material und mindestens einer elektrisch leitfähigen Kontaktfläche, die über ein Signalübertragungsmedium mit einem Signalsender und/oder Signalempfänger verbindbar ist und bei der Applikation den Nervus vagus an einem Teil seines Umfangs berührt.

Um während der Operation überprüfen zu können, ob möglicherweise im Operationsgebiet liegende Nervenstrukturen verletzt werden, wird schon seit mehreren Jahrzehnten bei Operationen ein intraoperatives Neuromonitoring durchgeführt. Die Technik des Neuromonitoring wird bei vielen chirurgischen Eingriffen, beispielsweise in der Neurochirurgie, der Hals-Nasen-Ohren-Chirurgie, der Gefäß- oder der Schilddrüsenchirurgie, eingesetzt. Dabei wird der zu überwachende Nerv elektrisch stimuliert und es kommt zur Ausbildung eines Aktionspotentials, das dann beispielsweise in Form von Elektromyographie-Signalen am Erfolgsorgan des betroffenen Nervs, zum Beispiel einem Muskel, über Elektroden abgeleitet und analysiert werden kann. Das Ableitsignal lässt auf den Funktionszustand des Nervs schließen.

Eine besondere Bedeutung hat das intraoperative Neuromonitoring unter anderem in der Schilddrüsenchirurgie, wo es zu einer Verletzung des Nervus laryngeus recurrens kommen kann, was zu Stimmstörungen, Verlust der Stimme und/oder Respirations- oder Schluckbeschwerden beim Patienten führt. Im Falle einer beidseitigen Verletzung des aus dem Nervus vagus abzweigenden Nervus laryngeus bei Strumaoperationen besteht für den Patienten sogar Lebensgefahr.

Durch den Einsatz der Funktionsüberwachung von Nerven bei der Operation konnte auch in der Schilddrüsenchirurgie die am häufigsten auftretende und gefährlichste Komplikation, nämlich die als Rekurrenzparese bezeichnete Verletzung des Nervus laryngeus recurrens signifikant gesenkt werden. Soweit ersichtlich, werden dabei bislang jedoch praktisch ausschließlich intermittierende Verfahren eingesetzt, bei denen eine Funktionskontrolle des Nervs nur vor und nach einer gefährlichen Tätigkeit des Operateurs durchgeführt werden kann, indem der Nerv mittels einer von Hand geführten Elektrode vor bzw. nach einem Schnitt oder dgl. angeregt wird. Die Stimulation erfolgt dabei über Stimulationssonden, deren konstruktive Gestaltung je nach Hersteller variiert. So kommen beispielsweise monopolare Sonden mit kugelförmigen Spitzen oder bipolare Ausführungen mit konzentrischen Polspitzen oder Gabelspitzen oder aber auch Hakensonden wahlweise mit geradem oder bajonettförmigem Schaft zum Einsatz. Mit allen diesen Stimulationssonden ist eine Stimulation des Nervs naturgemäß nur dann möglich, wenn sie vom Operateur von Hand an den zu stimulierenden Nerv angelegt wird.

Auch wenn das intermittierende Neuromonitoring sowohl für die Schilddrüsenchirurgie als auch bei anderen chirurgischen Eingriffen wie beispielsweise in der Neurochirurgie den Operateur bei der Identifikation eines gefährdeten Nervs sowie der Überprüfung der Nervenfunktion unterstützt, stellt dieses intermittierende Verfahren keinen sicheren Schutz für die gefährdeten Nerven dar, da ja immer nur vor und nach der an sich riskanten chirurgischen Tätigkeit eine Funktionsüberwachung stattfindet. Das intermittierende Monitoring hindert den Chirurgen also nicht daran, einen Nerv zu verletzen oder gar zu durchtrennen. Es wird daher ein kontinuierliches Überwachungsverfahren angestrebt, das als Art Frühwarnsystem für Nervenschädigungen angesehen werden kann. Unter dem Begriff 'kontinuierlich' soll in diesem Zusammenhang eine fortlaufende oder in kurzen zeitlichen Abständen selbständig, also ohne manuelle Tätigkeit des Operateurs erfolgende Stimulation des entsprechenden Nervs und die Ableitung des Antwortsignals am Erfolgsmuskel verstanden werden.

Anderseits eignen sich auch sogenannte Permanent-Elektroden, die zum dauerhaften Verbleib im menschlichen Körper vorgesehen sind, nicht zur intraoperativen Stimulation, da sie vergleichsweise schwer anzulegen und vor allem nur mit aufwendigen Eingriffen zu entfernen sind.

Aus der Druckschrift DE 10 2008 048 788 A1 der Anmelderin selbst ist eine Elektrode zur intraoperativen Nervenstimulation bekannt, die sich für ein kontinuierliches Neuromonitoring während einer Operation eignet, insbesondere für das Neuromonitoring des Nervus laryngeus recurrens bei Schilddrüsenoperationen. Die Elektrode ist gekennzeichnet durch einen Elektrodenkörper, der im Schnitt etwa T- oder ankerförmig ausgestaltet ist und einen Elektrodenschaft und mindestens einen von diesem beidseitig vorspringenden Haltebügel aufweist, wobei die Kontaktfläche an der dem Elektrodenschaft gegenüberliegenden Seite des Haltebügels angeordnet ist. Durch diese Ausbildung der Elektrode kann diese so zwischen der Vena jugularis interna und der Arteria carotis communis platziert werden, dass der Elektrodenschaft im Zwischenraum zwischen diesen beiden Blutgefäßen liegt und der Haltebügel mit seiner einen Seite an der Vene und mit seiner anderen Seite an der Arterie anliegt und mit seiner an der vom Elektrodenschaft abgewandten Vorderseite, an der sich die Kontaktfläche befindet, gegen den zu stimulierenden Nervus vagus drückt.

Diese Form einer Elektrode eignet sich zwar für eine intraoperative Nervenstimulation während einer Operation, weist aber hinsichtlich einer zuverlässigen Fixierung der Elektrode und einer sicheren Kontaktierung des Nervus vagus noch einige Schwächen auf. So ist durch die vorgegebene Form der Elektrode eine Anpassbarkeit an anatomische Varianzen nur im Rahmen der vorgeformten Kontur des T-förmig ausgestalteten Elektrodenkörpers möglich. Zudem kann die relativ kleine Kontaktfläche in Verbindung mit einer von den Blutgefäßen über die Haltebügel nur mäßig ausgeübten Anpresskraft nicht in jeder intraoperativen Situation eine zuverlässige elektrische Kontaktierung gewährleisten.

In der Druckschrift DE 10 2007 036 862 A1, die ebenfalls auf die Anmelderin zurückgeht, wurde eine Elektrode zur intraoperativen Stimulation des Nervus vagus vorgeschlagen, die gekennzeichnet ist durch einen um den zu stimulierenden Nerv herumschlingbaren, zu einer geschlossenen Schlinge schließbaren Kontaktstreifen aus elastischem, biokompatiblen Material, der mindestens eine dem Nerv zugewandte, elektrisch leitfähige Kontaktfläche aufweist, die mittels eines Signalübertragungsmittels mit einem Signalgeber und/oder einem Signalaufnehmer verbunden werden kann. Der Kontaktstreifen kann ähnlich wie ein Kabelbinder am zu stimulierenden Nerv appliziert werden und während der Operation als Manschette an diesem verbleiben und kontinuierlich mit Stimulationssignalen beaufschlagt werden.

Hierdurch wird zwar eine zuverlässige elektrische Kontaktierung des Nervus vagus erreicht, allerdings erfordert das An- und Ablegen der Elektrode viel Übung und Geschick vom Operateur, da der Vagusnerv im Operationsfeld verdeckt hinter den Blutgefäßen liegt und diese bei der zur Anlegung der Elektrode erforderlichen Freilegung des Nervs nicht verletzt werden dürfen. Auch darf der Kontaktstreifen nicht zu fest zusammengezogen werden, um eine Nervenquetschung zu vermeiden. Aus WO2007/021535 ist eine implantierbare Elektrode zur Stimulation des Nervus vagus bekannt, die den Nerv hakenförmig umschliesst, und mittels eines flexiblen Fortsatzes am Ösophagus fixierbar ist.

Die der vorliegenden Erfindung zu Grunde liegende Aufgabe besteht somit darin, eine Elektrode zum interoperativen Monitoring des Nervus vagus dahingehend zu verbessern, dass zum einen vor und nach der Operation ein unkompliziertes An- und Ablegen der Elektrode, zum anderen während des Eingriffs ein stabiler Sitz der Elektrode und eine zuverlässige Kontaktierung des Nervs gewährleistet sind.

Diese Aufgabe wird in Verbindung mit den Merkmalen des Oberbegriffs dadurch gelöst, dass der Elektrodenträger an seinem Kopfende einen ersten Teilabschnitt mit einem plastisch verformbaren Material aufweist, sodass bei der Applikation der Elektrodenträger mit dem ersten Teilabschnitt an eine Gefäßwand eines parallel zum Nervus vagus verlaufenden ersten Blutgefäßes anformbar ist und dieses Blutgefäß zur Fixierung der Elektrode hakenförmig umfasst und wobei der Elektrodenträger einen zweiten Teilabschnitt aufweist, der mit zum ersten Teilabschnitt gegenläufiger Krümmung schleifenartig um den Nervus vagus gelegt und zwischen dem ersten und einem zweiten parallel verlaufenden Blutgefäß hindurchgeführt werden kann.

Durch die Einarbeitung eines plastisch verformbaren Materials in einen am Kopfende des Elektrodenträgers liegenden ersten Teilabschnitt kann der Elektrodenträger formbar an die anatomische Struktur der Organe angepasst werden. In vorteilhafter Weise wird der Elektrodenträger in seinem Kopfbereich um das benachbarte Blutgefäß - die Arteria carotis communis - herum gebogen, sodass dieses entlang seines Umfangs hakenartig von dem Endabschnitt des Elektrodenträgers umfasst wird. Hierdurch erfährt die Elektrode einen sicheren Halt auch während der Operation.

In einem im mittleren bis fußseitigen Bereich des Elektrodenträgers liegenden zweiten Teilabschnitt wird der Elektrodenträger erfindungsgemäß mit gegenläufiger Krümmung zum ersten Teilabschnitt um den Nervus vagus herum geschlungen, wobei die dann innenliegende(n) Kontaktfläche(n) den Nerv berührt/berühren. Infolge der durch die hakenartige Aufhängung des Elektrodenträgers ausgeübten Zugkraft drückt die mindestens eine Kontaktfläche auf den Nervus vagus und gewährleistet eine zuverlässige Stimulation.

Im weiteren Verlauf zum Fußende hin wird der Elektrodenträger zwischen der Arteria carotis communis und einem zweiten Blutgefäß - der Vena jugularis interna-hindurchgeführt und dadurch an der der hakenförmigen Umfassung gegenüberliegenden Seite zusätzlich abgestützt.

Das Anlegen der Elektrode erfolgt also unkompliziert in der Weise, dass der Elektrodenträger von der Seite der Vena jugularis interna unter dem Nervus vagus durchgeführt und dann mit dem Elektrodenkopf um die Gefäßwand der Arteria carotis communis herumgelegt wird.

In der erfindungsgemäßen Lösung wird daher durch einen großen Umfangswinkel um die Arteria carotis communis eine hohe mechanische Stabilität sichergestellt und daraus resultierend in Verbindung mit einem ebenfalls großen Umfangswinkel um den Nervus vagus - bei entsprechender Ausgestaltung der Kontaktfläche - eine zuverlässige elektrische Kontaktierung ermöglicht.

In einer bevorzugten Ausgestaltung der Elektrode besteht das plastisch verformbare Material aus einer in den Elektrodenträger eingearbeiteten streifenförmigen Metallfolie. Durch die streifenförmige Metallfolie wird die plastische Verformbarkeit des Elektrodenkörpers zur Formung der hakenförmigen Halterung der Elektrode auf einfache Weise erreicht. Nach Einführung der Elektrode wird der Metallstreifen um die Gefäßwand der Arteria carotis communis gebogen und vermittelt so einen sicheren Halt, der nach erfolgter Operation wieder problemlos gelöst werden kann.

Um den durch die Umschlingung des Nervus vagus nutzbaren Kontaktbereich auszuschöpfen, erstreckt/erstrecken sich die Kontaktfläche(n) vorzugsweise streifenförmig parallel zur Längsausdehnung des Elektrodenträgers. Eine derartige Ausführung der Kontaktfläche(n) lässt auch bei kleinen Bewegungen der Elektrode eine zuverlässige kontinuierliche Stimulation zu.

Die Stimulation geschieht vorzugsweise über ein Verbindungskabel als Signalübertragungsmedium, welches an einem Fußende des Elektrodenträgers oder im Wesentlichen mittig zwischen der Biegung um das Blutgefäß und der Kontaktfläche zum Nervus vagus von dem Elektrodenträger abgeleitet wird. Eine leitungsgebundene Übertragung der Signale ist wenig anfällig gegenüber Funkfrequenzstörungen und trägt damit zur zuverlässigen Signalübertragung der Stimulationsimpulse bzw. deren Antwortsignalen bei. Das Verbindungskabel kann dabei je nach Operationsgegebenheiten am Fußende oder etwa mittig von dem Elektrodenträger abgeleitet werden.

Als vorteilhaft erweist sich, dass das elastische Trägermaterial, aus dem der Elektrodenträger besteht, elektrisch nicht leitendes medizinisches Silikon ist. In Verbindung mit einer als plastisch verformbares Material eingearbeiteten Metallfolie übernimmt das Trägermaterial die Funktion einer Isolierschicht für die Metallfolie gegenüber den Kontaktflächen. Die Metallfolie selbst wird vorzugsweise aus medizinischem Edelstahl hergestellt.

Eine vorteilhafte Weiterbildung der Elektrode kann einen größeren ersten Teilabschnitt mit eingearbeitetem plastisch verformbarem Material aufweisen, der sich in den zweiten Teilabschnitt hinein erstreckt. Ein derartig verlängerter Bereich plastisch verformbaren Materials ermöglicht eine noch stärke Fixierung der Elektrode, insbesondere im Bereich der Kontaktflächen. Dies wiederum erhöht die Zuverlässigkeit der Signalüberleitung von den Elektroden-Kontaktflächen auf den Nerv.

Des Weiteren wird die der Erfindung zu Grunde liegende Aufgabe in Verbindung mit den Merkmalen des Oberbegriffs auch dadurch gelöst, dass der Elektrodenträger zumindest an seinem Kopfende einen vorgeformten Abschnitt mit einer Biegung aufweist, sodass bei der Applikation der Elektrodenträger eine Gefäßwand eines parallel zum Nervus vagus verlaufenden ersten Blutgefäßes zur Fixierung der Elektrode hakenförmig umfasst und wobei der Elektrodenträger im weiteren Verlauf mit gegenläufiger Krümmung schleifenartig um den Nervus vagus gelegt und zwischen dem ersten und einem zweiten parallel verlaufenden Blutgefäß hindurchgeführt werden kann.

Im Unterschied zu der oben beanspruchten technischen Lehre besitzt der Elektrodenträger in dieser Lösung mindestens an seinem Kopfende einen vorgeformten Abschnitt mit einer Biegung, deren Krümmungsradius an dem Umfang der benachbart verlaufenden Arteria carotis communis bereits angepasst ist. Dieser gekrümmte vorgeformte Abschnitt wird bei Applikation hakenartig um die Gefäßwand der Arterie gelegt - gewissermaßen eingehakt - und fixiert so die eingeführte Elektrode. Im weiteren Verlauf wird der Elektrodenträger wie bei der oben beschriebenen Elektrode mit gegenläufiger Krümmung schleifenartig um den Nervus vagus herum geführt, so dass bei Ausgestaltung der Kontaktflächen als streifenförmig parallel zur Längsausdehnung des Elektrodenträgers verlaufende Leiterbahnen eine zuverlässige Kontaktierung gewährleistet ist. Zum Fußende hin wird der Elektrodenträger zwischen der Arteria carotis communis und der Vena jugularis interna in bekannter Weise gelegt und dadurch zusätzlich abgestützt.

Die Stimulation geschieht in gleicher Weise wie bei der Elektrode aus vollständig elastischem Material über ein Verbindungskabel mit endseitig oder mittig angeordneter Ableitung von dem Elektrodenträger.

Der vorgeformte Abschnitt kann aus einem im Wesentlichen starren Material bestehen und in bevorzugter Ausgestaltung als Spritzgussteil ausgeführt sein. Eine derartige Ausführung eignet sich in besonderem Maße für die industrielle Fertigung.

Weitere vorteilhafte Ausgestaltungsmerkmale ergeben sich aus der nachfolgenden Beschreibung und den Zeichnungen, die bevorzugte Ausführungsformen der Erfindung anhand von Beispielen erläutern. Dabei zeigen in schematischer Darstellung:
Fig. 1 eine erste Ausführungsform einer erfindungsgemäßen Elektrode mit langer Metallfolie in Draufsicht,
Fig. 2 den Gegenstand der Fig. 1 im Längsschnitt entlang der Schnittfläche II - II,
Fig. 3 eine zweite Ausführungsform der erfindungsgemäßen Elektrode mit kurzer Metallfolie in Draufsicht,
Fig. 4 eine dritte Ausführungsform der erfindungsgemäßen Elektrode mit mittig angeordneter Signalableitung in Draufsicht
Fig. 5 die zweite Ausführungsform der erfindungsgemäßen Elektrode bei Applikation in der Gefäß-Nemen-Scheide
Fig. 6 die dritte Ausführungsform der erfindungsgemäßen Elektrode bei Applikation in Gefäß-Nemen-Scheide
Fig. 7 eine Ausführungsform einer erfindungsgemäßen Elektrode mit einem vorgeformten Abschnitt bei Applikation in der Gefäß-Nemen-Scheide.

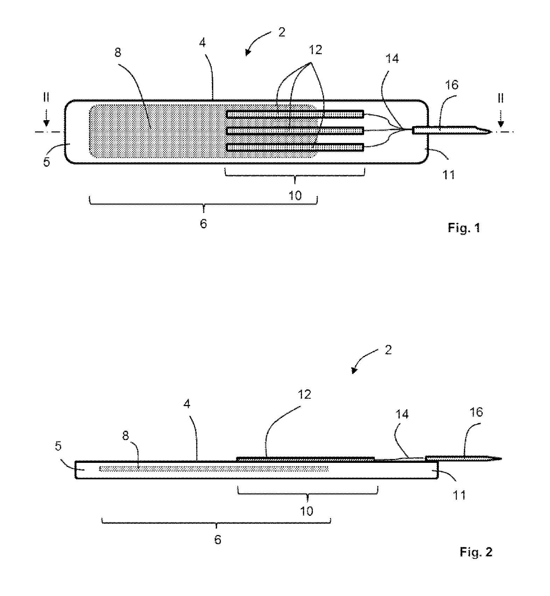

Fig. 1 gibt eine erste Ausführungsform einer erfindungsgemäßen Elektrode 2 wieder. In dieser Draufsicht ist als Grundelement der Elektrode 2 ein Elektrodenträger 4 zu sehen. Der Elektrodenträger 4 besteht aus elastischem Material, welches vorzugsweise elektrisch nicht leitendes medizinisches Silikon ist.

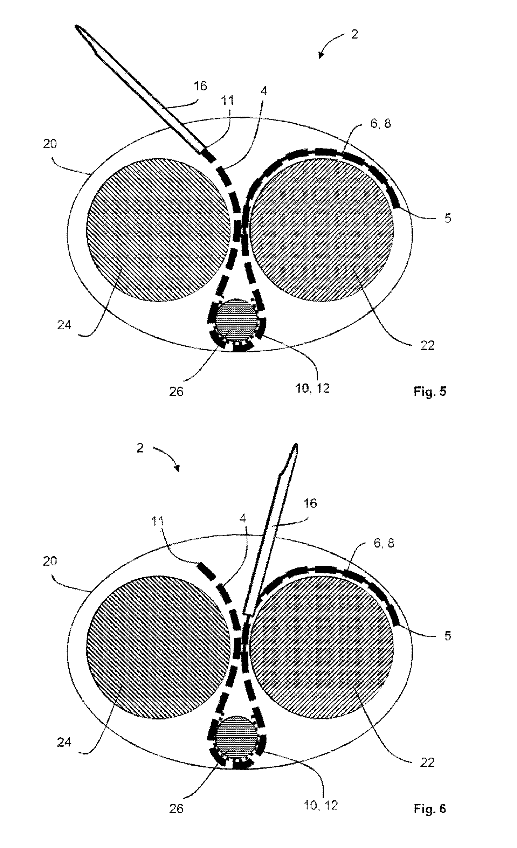

In dieses Trägermaterial 4 ist in einem dem Kopfende 5 des Elektrodenträgers 4 zugewandten ersten Teilabschnitt 6 ein plastisch verformbares Material in Form einer dünnen Metallfolie 8 eingebettet. Die Metallfolie 8 ist leicht formbar und kann somit in einfacher Weise an die anatomischen Strukturen der Körperorgane - hier an die Gefäßwand der Arteria carotis communis (siehe auch Fig. 5 und Fig. 6) - durch Biegung angepasst werden. Durch die Verwendung von elektrisch nicht leitendem medizinischem Silikon als Trägermaterial 4 erübrigt sich das Einziehen einer speziellen elektrischen Isolationsschicht, um die Metallfolie 8 von anderen Potential führenden elektrischen Komponenten der Elektrode zu trennen. Für die Metallfolie 8 erweist sich medizinischer Edelstahl als besonders geeignet.

Auf einer Außenseite des Elektrodenträgers 4 sind in einem zweiten Teilabschnitt 10 drei streifenförmige elektrische Kontaktflächen 12 angeordnet, die sich parallel zur Längsausdehnung des Elektrodenträgers 4 erstrecken. Der erste Teilabschnitt 6, in den die Metallfolie 8 eingearbeitet ist, erstreckt sich in den die Kontaktflächen 12 tragenden zweiten Teilabschnitt 10 hinein, sodass sich in dieser ersten Ausführungsform beide Teilabschnitte 6 und 10 überdecken.

Die Kontaktflächen 12 sind in Richtung des Fußendes 11 des Elektrodenträgers 4 mit Leitungsadern 14 verbunden, die das als Signalübertragungsmedium 16 dienende Verbindungskabel 16 bilden. Das Verbindungskabel 16 ist fußseitig 11 an dem Elektrodenträger 4 herausgeführt und an einen Signalsender und/oder einen Signalempfänger (nicht dargestellt) angeschlossen.

Fig. 2 zeigt die in Fig.1 dargestellte erste Ausführungsform im Längsschnitt entlang der Schnittfläche II - II. Deutlich zu erkennen ist, dass infolge der Überdeckung des ersten Teilabschnitts 6 mit dem zweiten Teilabschnitt 10 die in dem ersten Teilabschnitt 6 befindliche Metallfolie 8 unterhalb der in dem zweiten Teilabschnitt 10 gelegenen Kontaktflächen 12 angeordnet ist. Dabei umschließt der Elektrodenträger 4 aus elektrisch nicht leitendem medizinischem Silikon die eingebettete Metallfolie 8 und bildet eine elektrisch isolierende Trennschicht insbesondere zu den Kontaktflächen 12.

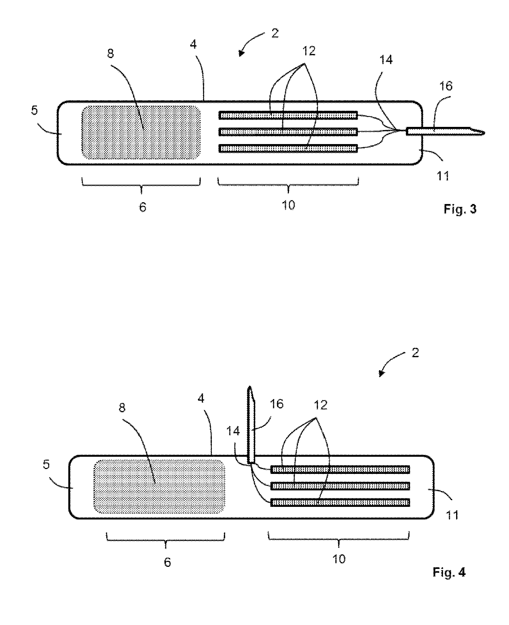

In Fig. 3 ist eine zweite Ausführungsform der erfindungsgemäßen Elektrode 2 zu sehen, bei der der erste Teilabschnitt 6, in den die Metallfolie 8 eingearbeitet ist, sich nicht in den die Kontaktflächen 12 tragenden zweiten Teilabschnitt 10 hinein erstreckt. Der Unterschied zu der ersten Ausführungsform besteht somit lediglich in einer verkürzten Länge der Metallfolie 8, die damit den plastisch verformbaren Teilabschnitt 6 auf einen im Wesentlichen die kopfseitige Hälfte des Elektrodenträgers 4 umfassenden Bereich beschränkt.

Eine weitere, dritte Ausführungsform der erfindungsgemäßen Elektrode 2 ist in Fig. 4 abgebildet. In dieser Ausführungsform weist die Metallfolie 8 ebenso wie die in Fig. 3 offenbarte Elektrode 2 eine verkürzte Länge auf, allerdings werden in dieser dritten Ausführungsform die Leitungsadern 14 und damit das Verbindungskabel 16 nicht an dem Fußende 11 des Elektrodenträgers 4 abgeleitet. Stattdessen ist die Verbindung zwischen Kontaktflächen 12 und dem Verbindungskabel 16 zur Mitte des Elektrodenträgers 4 hin ausgerichtet. Diese Ausführungsform kann besonderen räumlichen Gegebenheiten während der Operation Rechnung tragen. Bis auf die Ausrichtung der Kontaktflächen 12 entspricht diese Ausführungsform im Übrigen der in Fig. 3 beschriebenen Ausführung einer Elektrode 2 mit kurzer eingearbeiteter Metallfolie 8.

Die Fig. 5 bis 7 beziehen sich auf die Applikation der erfindungsgemäßen Elektrode 2 bei einer Schilddrüsenoperation. Schematisch dargestellt ist eine jeweilige Ausführungsform der Elektrode 2 in Verbindung mit der Anatomie der Gefäß-Nerven-Scheide 20 (Vagina carotica). Veranschaulicht ist jeweils auf der rechten Körperseite schädelwärts blickend die Lage der Arteria carotis communis 22, der Vena jugularis interna 24 sowie des zu stimulierenden Nervus vagus 26.

Fig. 5 zeigt mit Bezug auf Fig. 3 die Position der Elektrode 2 mit verkürzter Länge der Metallfolie 8 bei einem operativen Eingriff. Beim Anlegen führt der Operateur die Elektrode 2 von der Seite der Vena jugularis interna 24 unter dem Nervus vagus 26 hindurch, um sie dann auf der Arteria carotis communis 22 hakenförmig abzustützen. Hierbei ergibt sich eine zur Krümmung im ersten Teilabschnitt 6 gegenläufig gekrümmte schleifenartige Umfassung des Nervus vagus 26 durch den die Kontaktflächen 12 tragenden zweiten Teilabschnitt 10 des Elektrodenträgers 4. Der große Umfassungswinkel gewährleistet in Verbindung mit den streifenförmig parallel zur Längsausdehnung des Elektrodenträgers 4 angeordneten Kontaktflächen 12 eine zuverlässige Kontaktierung des zu stimulierenden Nervs 26. Die Abstützung auf der Arteria carotis communis 22 erfolgt durch Formung einer Biegung aus der im Bereich des ersten Teilabschnitts 6 in den Elektrodenträger 4 eingebetteten Metallfolie 8. So kann der Elektrodenträger 4 an seinem Kopfende 5 an die Gefäßwand der Arteria carotis communis 22 plastisch angepasst werden. Die Ableitung des Verbindungskabels 16 erfolgt entsprechend Fig. 3 am fußseitigen Ende 11 des Elektrodenträgers 4.

In Fig. 6 ist die dritte Ausführungsform gemäß Fig. 4 mit kurzer Metallfolie 8 und mittig liegender Ableitung des Verbindungskabels 16 dargestellt. Je nach Operationsumgebung - z.B. bei beengten Raumverhältnissen im Bereich der Vena jugularis interna 24 - kann eine derartige mittige Lage der Kabelableitung zwischen Stimulationsfläche und Biegung um die Arteria carotis communis 22 vorteilhaft sein.

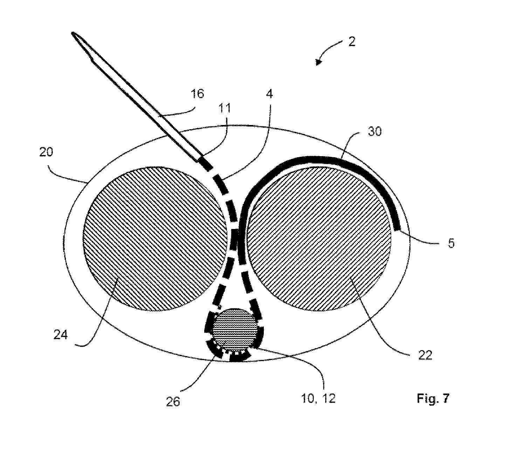

In Fig. 7 wird die Lage einer weiteren erfindungsgemäßen Elektrode 2 dargestellt, bei der der Elektrodenträger 4 an seinem Kopfende 5 einen vorgeformten Abschnitt 30 mit einer Biegung aufweist, deren Krümmungsradius an dem Umfang der Arteria carotis communis 22 angepasst ist. Die Elektrode 2 kann wie eine Elektrode, die vollständig aus elastischem Material besteht, in die Gefäß-Nemen-Scheide 20 eingeführt und schleifenartig um den Nervus vagus 26 herum geführt werden. Gekennzeichnet ist diese Elektrode 2 dadurch, dass der gekrümmte Abschnitt 30, mit dem die Elektrode 2 an ihrem Kopfende 5 an der Arteria carotis communis 22 'eingehakt' wird, aus einem biegeelastischen Material bereits vorgeformt ist. Dabei kann sich der vorgeformte Bereich über die Biegung um die Arteria carotis communis 22 hinaus in den die Kontaktflächen 12 tragenden zweiten Teilabschnitt 10 hinein erstrecken.

Mit der Erfindung wird eine Elektrode zur intraoperativen Stimulation des Nervus Vagus geschaffen, mit einem mindestens eine Kontaktfläche aufweisenden, etwa streifenförmig ausgebildeten Elektrodenträger, der so an dem Nervus Vagus applizierbar ist, dass die Kontaktfläche den Nerv wenigstens an einem Teil seines Umfangs berührt, wobei die Ausgestaltung erfindungsgemäß so getroffen ist, dass der Elektrodenträger einen ersten gekrümmten Abschnitt aufweist, der an den Nervus Vagus angepasst oder anpassbar ist und an dessen Innenseite die mindestens eine Kontaktfläche angeordnet ist, und dass der Elektrodenträger midestens einen zweiten Abschnitt aufweist, der gegenläufig zur Krümmung des ersten Abschnitts etwa hakenartig geformt oder formbar ist und an ein zum Vagusverv benachbart verlaufendes Blutgefäß angepasst oder anpassbar ist.

Die Erfindung ist nicht auf die dargestellten Ausführungsbeispiele beschränkt, sondern es ergeben sich verschiedene Änderungen oder Ergänzungen, ohne den Rahmen der Erfindung zu verlassen. So kann die gesamte Elektrode 2 aus einem vorgeformten Teil hergestellt sein, wobei es bevorzugt aus einem biegelastischen Material besteht, welches gemäß den Fig. 5 bis 7 in seiner Formgebung an die Anatomie der Gefäß-Nerven-Scheide 20 angepasst ist und den dargestellten vorteilhaften Krümmungsverlauf aufweist, bei Applikation der Elektrode am Vagusnerv sich aber verbiegen und dadurch leicht anlegen und auch wieder entfernen lässt.

## Patentansprüche

1. Elektrode zur intraoperativen Stimulation des Nervus vagus (26) mit einem streifenförmig ausgebildeten Elektrodenträger (4) und mindestens einer elektrisch leitfähigen Kontaktfläche (12), die über ein Signalübertragungsmedium (16) mit einem Signalsender und/oder Signalempfänger verbindbar ist und bei der Applikation den Nervus vagus (26) an einem Teil seines Umfangs berührt, **dadurch gekennzeichnet, dass** der Elektrodenträger (4) einen ersten, plastisch verformbaren Teilabschnitt (6) aufweist, der bei Applikation des Elektrodenträgers (4) an eine Gefäßwand eines parallel zum Nervus vagus (26) verlaufenden ersten Blutgefäßes (22) anformbar ist und dieses Blutgefäß (22) zur Fixierung der Elektrode (2) hakenförmig umfasst und dass der Elektrodenträger (4) einen zweiten Teilabschnitt (10) aufweist, der mit zum ersten Teilabschnitt (6) gegenläufiger Krümmung schleifenartig um den Nervus vagus (26) gelegt und zwischen dem ersten und einem zweiten parallel verlaufenden Blutgefäß (24) hindurchgeführt werden kann.

2. Elektrode nach Anspruch 1, **dadurch gekennzeichnet, dass** der plastisch verformbare Teilabschnitt (6) eine in den Elektrodenträger eingearbeitete, streifenförmige Metallfolie (8) aufweist.

3. Elektrode nach Anspruch 1 oder 2, **dadurch gekennzeichnet, dass** der Elektrodenträger (4) aus elektrisch nicht leitendem medizinischen Silikon besteht.

4. Elektrode nach Anspruch 2 oder 3, **dadurch gekennzeichnet, dass** die Metallfolie (8) aus medizinischem Edelstahl besteht.

5. Elektrode nach einem der Ansprüche 1 bis 4, **dadurch gekennzeichnet, dass** sich der plastisch verformbare erste Teilabschnitt (6) mindestens bis zur Kontaktfläche (12) erstreckt.

6. Elektrode zur intraoperativen Stimulation des Nervus vagus (26) mit einem streifenförmig ausgebildeten Elektrodenträger (4) aus elastischem Material und mindestens einer elektrisch leitfähigen Kontaktfläche (12), die über ein Signalübertragungsmedium (16) mit einem Signalsender und/oder Signalempfänger verbindbar ist und bei der Applikation den Nervus vagus (26) an einem Teil seines Umfangs berührt, **dadurch gekennzeichnet, dass** der Elektrodenträger einen ersten etwa hakenförmig geformten Abschnitt (30) mit einer Biegung aufweist, der bei der Applikation der Elektrode eine Gefäßwand eines parallel zum Nervus vagus (26) verlaufenden ersten Blutgefäßes (22) zur Fixierung der Elektrode (2) übergreift, und dass der Elektrodenträger (4) einen zweiten Abschnitt mit gegenläufiger Krümmung aufweist, mit der er schleifenartig um den Nervus vagus (26) gelegt und zwischen dem ersten und einem zweiten parallel verlaufenden Blutgefäß (24) hindurchgeführt werden kann.

7. Elektrode nach einem der Ansprüche 1 bis 6, **dadurch gekennzeichnet, dass** sich die Kontaktfläche(n) (12) streifenförmig parallel zur Längsausdehnung des Elektrodenträgers (4) erstreckt/erstrecken.

8. Elektrode nach einem der Ansprüche 1 bis 7, **dadurch gekennzeichnet, dass** das Signalübertragungsmedium (16) ein Verbindungskabel (16) ist, welches an einem Ende (11) des Elektrodenträgers (4) oder im Übergangsbereich zwischen der Biegung um das erste Blutgefäß (22) und der Kontaktfläche zum Nervus vagus (26) von dem Elektrodenträger (4) abgeleitet wird.

9. Elektrode nach einem der Ansprüche 6 bis 8, **dadurch gekennzeichnet, dass** der Elektrodenträger (4) ein Spritzgussteil ist und bevorzugt aus einem biegeelastischen Material besteht.

## Claims

1. Electrode for intraoperatively stimulating the vagus nerve (26), comprising a strip-shaped electrode holder (4) and at least one electrically conductive contact surface (12) which is connectable via a signal transmission medium (16) to a signal transmitter and/or signal receiver and which, during application, touches part of the circumference of the vagus nerve (26), **characterised in that** the electrode holder (4) comprises a first, plastically deformable sub-portion (6), which, during application of the electrode holder (4), can be integrally formed onto a vessel wall of a first blood vessel (22) extending in parallel with the vagus nerve (26) and encompasses said blood vessel (22) in order to secure the electrode (2) in a hook-shaped manner, and **in that** the electrode holder (4) comprises a second sub-portion (10), which, with a curvature in the opposite direction to the first sub-portion (6), can be placed around the vagus nerve (26) in a loop-like manner and can be passed through between the first and a second parallel blood vessel (24).

2. Electrode according to claim 1, **characterised in that** the plastically deformable sub-portion (6) has a strip-shaped metal foil (8) incorporated in the electrode holder.

3. Electrode according to either claim 1 or claim 2, **characterised in that** the electrode holder (4) consists of electrically non-conductive medical silicone.

4. Electrode according to either claim 2 or claim 3, **characterised in that** the metal foil (8) consists of medical stainless steel.

5. Electrode according to any of claims 1 to 4, **characterised in that** the plastically deformable first sub-portion (6) extends at least as far as the contact surface (12).

6. Electrode for intraoperatively stimulating the vagus nerve (26), comprising a strip-shaped electrode holder (4) made of a resilient material, and at least one electrically conductive contact surface (12) which is connectable via a signal transmission medium (16) to a signal transmitter and/or signal receiver and which, during application, touches part of the circumference of the vagus nerve (26), **characterised in that** the electrode holder has a first, almost hook-shaped portion (30) which has a bend and which, during application of the electrode, extends over a vessel wall of a first blood vessel (22) extending in parallel with the vagus nerve (26) in order to secure the electrode (2), and **in that** the electrode holder (4) comprises a second portion having a curvature in the opposite direction to said first portion, by means of which curvature the second portion can be placed around the vagus nerve (26) in a loop-like manner and passed through between the first and a second parallel blood vessel (24).

7. Electrode according to any of claims 1 to 6, **characterised in that** the contact surface(s) (12) extend(s) in parallel with the longitudinal extent of the electrode holder (4) in a strip-shaped manner.

8. Electrode according to any of claims 1 to 7, **characterised in that** the signal transmission medium (16) is a connection cable (16) which is routed from the electrode holder (4), at one end (11) of the electrode holder (4) or in the transition region between the bend around the first blood vessel (22) and the contact surface, to the vagus nerve (26).

9. Electrode according to any of claims 6 to 8, **characterised in that** the electrode holder (4) is an injection-moulded part and preferably consists of a flexurally elastic material.

## Revendications

1. Electrode pour la stimulation peropératoire du nerf vague (26) avec un porte-électrode (4) conformé en bande et au moins une surface de contact conductrice de l'électricité (12), qui peut être connectée, via un support de transmission de signal (16), à un émetteur de signal et/ou à un récepteur de signal et qui, lors de l'application, vient en contact avec le nerf vague (26) sur une partie de sa périphérie, **caractérisée en ce que** le porte-électrode (4) présente une première section partielle (6) à déformation plastique, qui, lors de l'application du porte-électrode (4), peut être adaptée à une paroi vasculaire d'un premier vaisseau sanguin s'étendant parallèlement au nerf vague (26) et enserre ce vaisseau sanguin (22) en forme de crochet pour fixer l'électrode (2) et **en ce que** le porte-électrode (4) présente une seconde section partielle (10) qui, avec une courbure opposée à celle de la première section partielle (6), peut être disposée en forme de boucle autour du nerf vague (26) et s'étendre entre le premier et un second vaisseau sanguin (24) s'étendant parallèlement.

2. Electrode selon la revendication 1, **caractérisée en ce que** la section transversale à déformation plastique (6) présente un film métallique en forme de bande (8) incorporé au porte-électrode.

3. Electrode selon la revendication 1 ou la revendication 2, **caractérisée en ce que** le porte-électrode (4) est constitué de silicium médical non conducteur de l'électricité.

4. Electrode selon la revendication 2 ou la revendication 3, **caractérisée en ce que** le film métallique (8) est constitué d'acier fin médical.

5. Electrode selon l'une quelconque des revendications 1 à 4, **caractérisée en ce que** la première section partielle à déformation plastique (6) s'étend au moins jusqu'à la surface de contact (12).

6. Electrode pour la stimulation peropératoire du nerf vague (26), comprenant un porte-électrode (4) conformé en bande (4) formé d'un matériau élastique et au moins une surface de contact conductrice de l'électricité (12), qui peut être connectée via un support de transmission de signal à un émetteur de signal et/ou à un récepteur de signal et vient, lors de l'application, en contact avec le nerf vague sur une partie de sa périphérie, **caractérisée en ce que** le porte-électrode présente une première section (30) conformée approximativement en crochet avec une courbure qui, lors de l'application de l'électrode, recouvre une paroi vasculaire d'un premier vaisseau sanguin (22) s'étendant parallèlement au nerf vague (26) pour fixer l'électrode (2) et **en ce que** le porte-électrode (4) présente une seconde section à courbure opposée avec laquelle il peut être disposé en forme de boucle autour du nerf vague (25) et s'étendre entre le premier et un second vaisseau sanguin (24) s'étendant parallèlement.

7. Electrode selon l'une quelconque des revendications 1 à 6, **caractérisée en ce que** la ou les surfaces de contact (12) s'étend ent) en forme de boucle parallèlement à l'extension longitudinale du porte-électrode (4).

8. Electrode selon l'une quelconque des revendications 1 à 7, **caractérisée en ce que** le support de transmission de signal (16) est un câble de liaison (16) qui est dérivé du porte-électrode (4) à une extrémité (11) du porte-électrode (4) ou dans la zone de transition entre la courbure autour du premier vaisseau sanguin (22) et la surface de contact avec le nerf vague (26).

9. Electrode selon l'une quelconque des revendications 6 à 8, **caractérisée en ce que** le porte-électrode (4) est une pièce moulée par injection et est de préférence constitué d'un matériau élastique à la flexion.
